# EUROPEAN PATENT SPECIFICATION

(11) **EP 1 586 579 B1**
(45) Date of publication and mention of the grant of the patent: **22.04.2015**
(21) Application number: 05101435.5
(22) Date of filing: 25.02.2005
(51) Int. Cl.: C07J 1/00, C07J 21/00, C07J 31/00

(54) **Process for the preparation of delta(15-16)-17-ketosteroids and use thereof in the synthesis of pharmacologically active compounds**
Verfahren zur Herstellung von Delta(15,16)-17-ketosteroiden und deren Verwendung bei der Synthese pharmakologisch aktiver Verbindungen
Procédé de préparation de composés 17-cétostéroides et leur utilisation dans la synthèse de composés à activité pharmacologique

(30) Priority: 08.03.2004 IT MI20040443
(43) Date of publication of application: 19.10.2005
(73) Proprietor: Euticals S.P.A., 20122 Milan (IT)
(72) Inventor: Grisenti, Paride, I-20141, Milano (IT); Reza Helai, Sharzad, I-20068, Peschiera Borromeo (MI) (IT); Pengo, Daniele, I-20037, Paderno Dugnano (MI) (IT)
(74) Representative: Pistolesi, Roberto

(56) References cited:
- EP-A- 0 700 926
- DE-A- 2 546 062
- DE-A- 2 630 419
- P. FERRABOSCHI ET AL.: "A Simple Synthesis of Gestodene from 18-Methyl-4-estren-3,17-dion" SYNLETT, no. 10, 29 June 2004 (2004-06-29), pages 1838-1840, XP002335476
- DATABASE BEILSTEIN [Online] BEILSTEIN INSTITUT ZUR FÖRDERUNG DER WISSENSCHAFTEN; 27 June 1998 (1998-06-27), XP002335480 Database accession no. 3270683,3271515 & G. GROSZEK ET AL.: BULL. POL. ACAD. SCI. CHEM., vol. 34, no. 7-8, 1986, pages 305-311,
- B. M. TROST ET AL.: "New Synthetic Reactions. Sulfenylations and Dehydrosulfenylations of Esters and Ketones" J. AM. CHEM. SOC., vol. 98, no. 16, 4 August 1976 (1976-08-04), pages 4887-4902, XP002335477
- R. CAPUTO ET AL.: "Thiosulfonic S-Esters; 6. Fluoride-Mediated .alpha.-Phenylsulfenylation of Cyclic Ketones and Esters via their Trimethylsilyl Enol Ethers" SYNTHESIS, 1989, pages 464-466, XP002335478
- W. G. DAUBEN ET AL.: "A Formal Total Synthesis of Fusidic Acid" J. AM. CHEM. SOC., vol. 104, no. 1, 1982, pages 303-305, XP002335479
- B. M. TROST: ".alpha.-Sulfenylated Carbonyl Compounds in Organic Synthesis", CHEM. REV., vol. 78, no. 4, 1978, pages 363-382,

## Description

Gestodene (17-α-ethynyl-17-β-hydroxy-18-methyl-4,15-estradien-3-one, Figure 1) of the formula 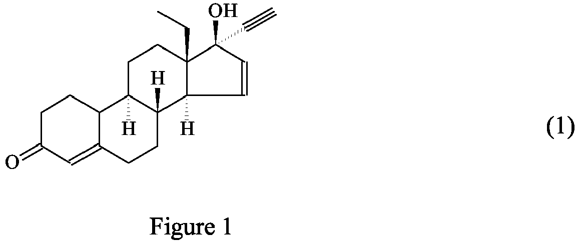 is a synthetic progestin used in hormone replacement therapy and, in combination with an oestrogen, as an oral contraceptive.

This molecule, described for the first time by H. Hofmeister in 1975 (DE2546062) is a third generation progestin with low androgenic activity and probably reduced side effects relative to second generation progestins, such as for example levonorgestrel (J. Obstet. Gynecol. 15:195-200;1995). There are many process patents which describe the synthesis of this molecule (DE2546062; DE2636404; DE2636407; DE2749104; EP201452; EP700926) and which specify 18-methyl-4-estren-3,17-dione (2a) as the starting compound; these patents use a chemical/microbiological approach. Starting from 18-methyl-4-estren-3,17-dione (2a), which is commercially available, the crucial step for the synthesis of gestodene, and in general of its Δ¹⁵⁻¹⁶, analogues is precisely the introduction of the double bond in this specific position.

This double bond is generally introduced by means of an elimination reaction performed on a derivative which is hydroxylated in position 15, which was itself prepared by a microbiological transformation of 18-methyl-4 estren-3,17 dione (2a) (US4,081,537); the stated yields for this microbiological step, which provides the use of *Penicillium raistrickii* ATCC10490, are 76% (scheme 1):

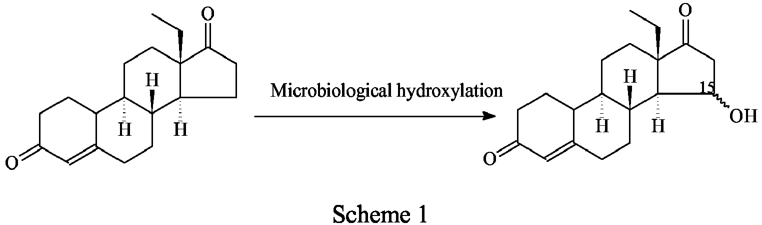

Data from literature published subsequently to the cited patent US4,081,537 describe this microbiological step as being much less than satisfactory with regard both to bioconversion yields and to recovery and purification yields for the hydroxylated product from the fermentation medium (Journal of Basic Microbiology (1991), 31(5), 3 85-90.; Mededelingen van de Faculteit Landbouwwetenschappen, University of Ghent (1991), 56(4b), 1785-7.; Folia Microbiologica (Prague, Czech Republic) (1992), 37(4), 249-55.; Acta Biotechnologica (1995), 15(2), 161-71; Journal of Molecular Catalysis B: Enzymatic (1998), 5(1-4), 385-387; Huaxue Xuebao (2001), 59(4), 604-609).

According to the process described in patent US4,081,537 and shown in the following scheme: 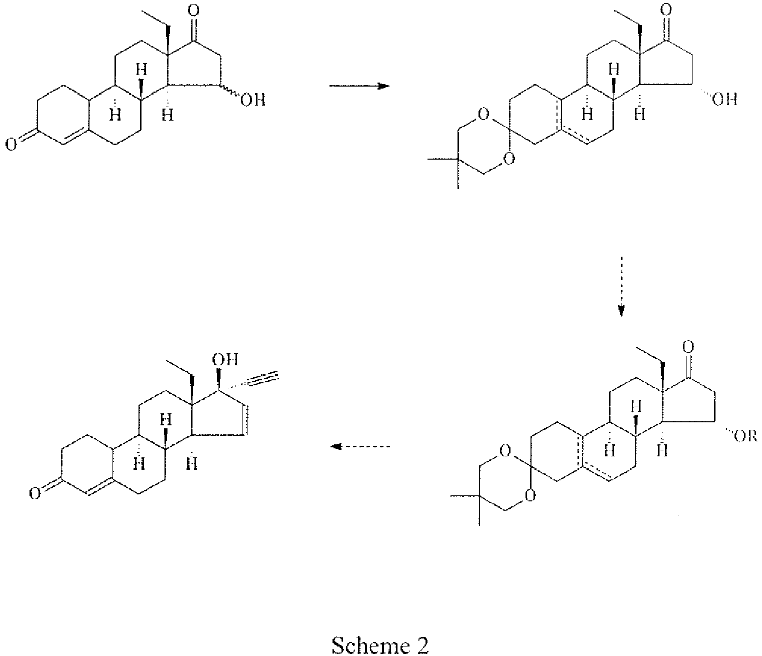 this hydroxylated intermediate (15-α-hydroxy-18-methyl-4-estren-3,17-dione) is subsequently converted into the desired product by means of a reaction sequence which provides, inter alia, the protection of the ketone in position 3 as a ketal, esterification of the hydroxyl in position 15 and ethynylation of the ketone in position 17. Subsequent acid hydrolysis of the crude reaction product yields gestodene in overall process yields of 21% starting from 18-methyl-4-estren-3,17-dione (2a).

J. Am. Chem. Soc 1982, 104(1), 303-305 also describes the protection of the ketone in position 3 of an analogue of 3-keto-androstan-15-one by an ethylketal.

Androst-15-en-17-one derivatives can be prepared starting from their androstan-17-one analogues via sulfinylation with methylbenzenesulfinate and sodium hydride, and then submitted to pyrolysis in xylene with N,N-dimethylaniline (Bull. Pol. Acad. Sci. Chem. 1986, 34(7-8), 305-311).

B. M. Trost et al. describes in Chem. Rev. 1978, 78(4), 363-382) that the corresponding phenyl sulfide of estrone methyl ether can be obtained through a sulfanylation reaction with diphenyl disulfide, which is subsequently oxidised with meta-chloro perbenzoic acid (mCPBA) to provide the desired unsaturated compound.

EP700926 discloses the ethynylation reaction of a 3-ketal-androst-5,17-dien-15-one by Li acetylide and subsequent hydrolysis to give a androst-4,17-dien-3,15-dione core.

Another critical point of this synthetic pathway resides in the fact that the protective group in position 3 results in displacement of the double bond which was initially in position Δ⁴ to give rise to a mixture of protected intermediates in which the double bond is located 50% in position 5-6 and 50% in position 5-10. As a result, these synthetic intermediates cannot be isolated as crystalline solids, but in oily form and may thus only be purified by chromatography.

Literature published subsequently to that cited (EP201452 and Arzneim.-Forsch./Drug Res. 36 (1) 781-783, 1986) describes an alternative synthetic pathway for gestodene, again starting from 15-α-hydroxy-4-estren-3,17-dione, which provides protecting the ketone in position 3 as a methylenol ether and thus introducing the ethynyl in position 17. The resultant intermediate is not isolated from the reaction mixture but treated in an acidic medium, making it possible, in a single step, to re-establish the 3-keto-Δ⁴ system and to introduce the double bond in position 15-16.

This method makes it possible to obtain gestodene with overall yields of 47% (starting from 18-methyl-4-estren-3,17-dione (2a)) as shown in the following scheme:

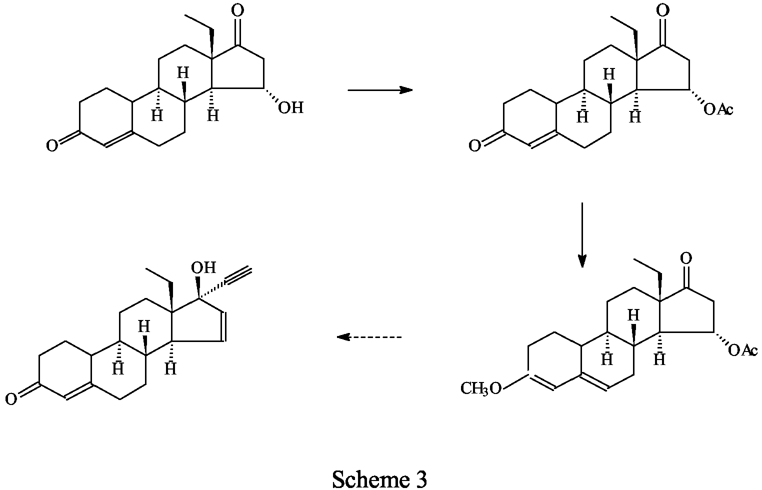

However, when replicated experimentally, the above-described method does not give rise to the stated reaction yields, in that, apart from gestodene, two secondary products are formed in a quantity of greater than 10%. Since every attempt to purify the resultant product by crystallisation proved ineffective, we used chromatographic methods and so obtained pure gestodene, but at the expense of overall process yields and making use of a purification method which is always disadvantageous on an industrial scale. Another possible method of introducing the double bond in position 15-16 of the steroid ring D is proposed by McMorris et al. (Tetrahedron 46, 7, 2287-2306, 1990) and substantially provides introducing bromine in position 16 of a 17-β-ketal. However, this synthetic pathway has proved rather unsatisfactory in our case, both due to the laborious conversion required by the 3-keto-Δ⁴ system present in the ring A, and because, during removal of the ketone in position 17 in an acidic medium, a partial migration of the double bond from position 15-16 to position 14-15 also occurs.

One possible alternative method for forming the double bond 15-16 which we have tested, is reaction with diphenyl diselenide as described in Steroids 61, 74-81, (1996); unfortunately, this method too has proved unsatisfactory in that, under the described conditions, it is not possible to obtain the corresponding 16-phenyl selenide derivative.

In conclusion, in the light of what has been verified experimentally, currently known methods for the preparation of Δ¹⁵¹⁶-17-ketosteroids and, in particular, of gestodene are not at all satisfactory, at least in terms of yield and experimental complications.

We have, however, surprisingly found that is possible to prepare Δ¹⁵⁻¹⁶-17-ketosteroids and, in particular gestodene, by means of an exclusively chemical, readily industrially applicable process which makes it possible, starting from the same substrates, to avoid the laborious microbiological hydroxylation step and to achieve a considerable improvement in overall process yields, without having to make use of particularly hazardous reactants.

### Description of the invention

The present invention accordingly provides a process for the preparation of Δ¹⁵¹⁶-17-ketosteroids of the formula 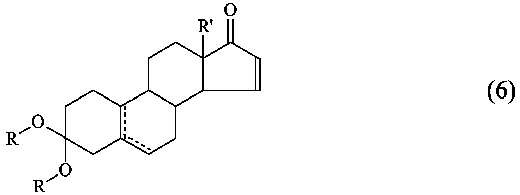 in which
R' represents hydrogen, methyl or ethyl,
the two instances of R together represent a divalent group -(CH₂)₂-, - (CH₂)₃-, -CH₂-C(CH₃)₂-CH₂- , and
the dashed line represents a 5-6 or 5-10 double bond, or a mixture thereof, which comprises:
   a) the reaction of a 17-ketosteroid of the formula 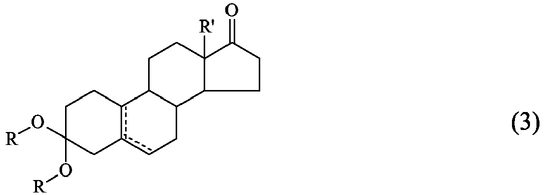 in which R, R' and the dashed line have the above-stated meanings, with diphenyl disulfide in the presence of a strong base
      to yield the compound of the formula 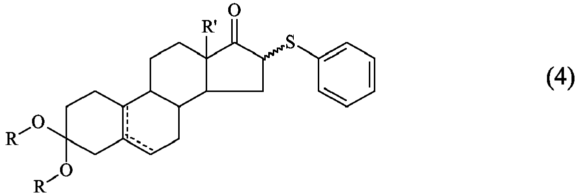 in which R, R' and the dashed line have the above-stated meanings,
   b) the subsequent oxidation reaction of the compound of the formula (4) to yield the compound of the formula 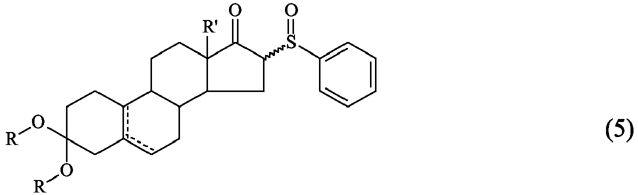 in which R, R' and the dashed line have the above-stated meanings, and
   c) the elimination reaction of the compound of the formula (5) to yield the Δ¹⁵⁻¹⁶-17-ketosteroid compound of the formula (6).

A preferred embodiment of the above-described process comprises:
a) the reaction of a 17-ketosteroid of the formula 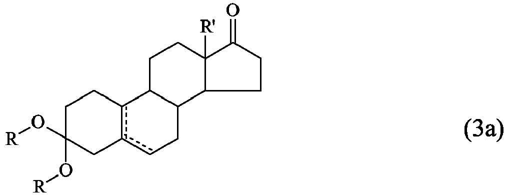 in which the two groups R together represent -CH₂-C(CH₃)₂-CH₂- , R' is ethyl and the dashed line has the above-stated meaning,
   with diphenyl disulfide in the presence of a strong base
   to yield the compound of the formula 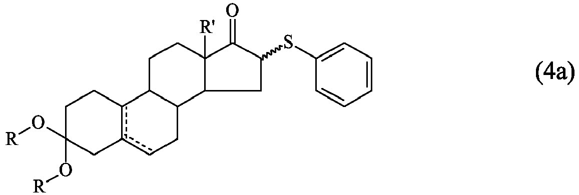 in which R, R' and the dashed line have the above-stated meanings,
b) the subsequent oxidation reaction of the compound of the formula (4) to yield the compound of the formula 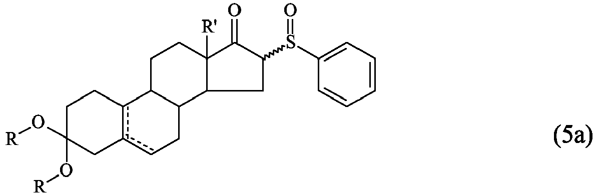 in which R, R' and the dashed line have the above-stated meanings, and
c) the elimination reaction of the compound of the formula (5a) to yield the ^{Δ}15-16-17-ketosteroid compound of the formula 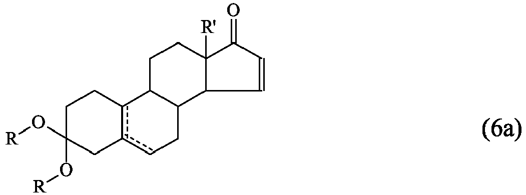 in which R, R' and the dashed line have the above-stated meanings.

This latter process is particularly preferred in that it makes it possible to prepare gestodene (1) with good yields and without making use of microbiological type reactions in accordance with the following general synthetic scheme:

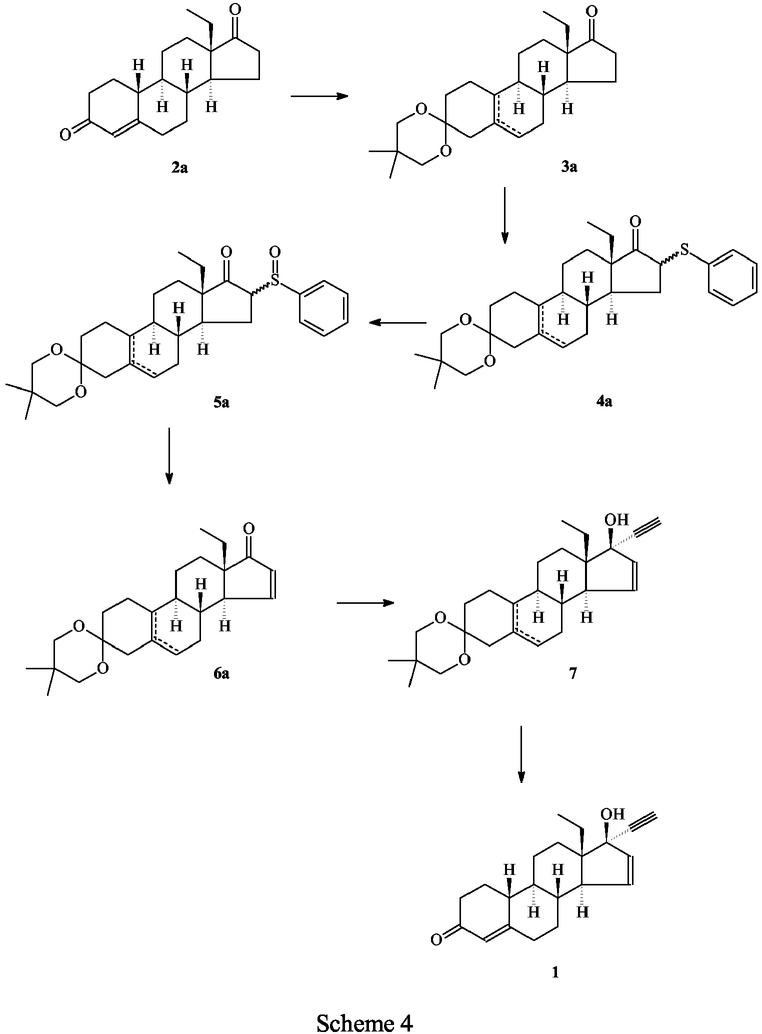

The compound of the formula (3) is generally prepared by selective ketalisation in position 3 of the corresponding 3,17-diketosteroid of the formula 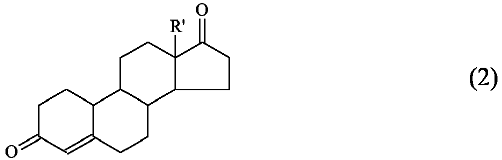 in which R' represents hydrogen, methyl or ethyl, preferably ethyl.

The compounds of the formula (2) are known and are commercially available, for example from the suppliers Kingchem Fine Chemicals; Beecham Product List, SIGMA or Keifeng SNC.

Said selective protection of the ketone group in position 3 is generally performed under conventional conditions for the reaction of the diketone of the formula (2) with a diol of the formula HO-(CH₂)₂-OH, HO-(CH₂)₃-OH, HO-CH₂-C(CH₃)₂-CH₂-OH, in the presence of a suitable acid catalyst. In a preferred embodiment, the ketal of the formula 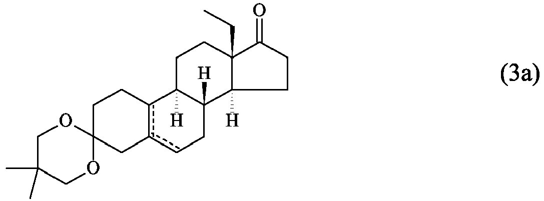 is prepared by treating 18-methyl-4-estren-3,17-dione (2a) with 2,2-dimethylpropanediol and triethyl orthoformate in the presence of an acid catalyst, preferably p-toluenesulfonic acid, preferably using dichloromethane as solvent to yield the corresponding ketal (3a) with yields of around 90%. This intermediate is present as an 80/20 mixture of Δ⁵ and Δ⁵⁻¹⁰ isomers and was used as it was for the subsequent functionalisation reactions in position 16.

The compounds of the formula (3a), in which two groups R together represent -CH₂-C(CH₃)₂-CH₂-, R' is ethyl and the double bond is in position 5-6 or 5-10, namely 18-methyl-3,3-(2',2'-dimethyl-1',3'-propylenedioxy)-5-estren-17-one and 18-methyl-3,3-(2',2'-dimethyl-1',3'-propylenedioxy)-5(10)-estren-17-one are new and have been synthesised by us for the first time.

While these products are indeed indexed in Chemical Abstracts under register numbers 64133-04-6 and 64133-03-5, they are not described in the only cited literature reference (DE2546062). This document in fact only presents steroidal analogues which are microbiologically hydroxylated in position 15. Step a) of the process provided by the present invention, which is all the more surprising when account is taken of the negative results previously obtained with the analogous bromination or diphenyl diselenide functionalisation reactions, is preferably performed with diphenyl disulfide and in the presence of a strong base, preferably selected from among potassium t-butylate, sodium t-butylate, sodium hydride, n-butyllithium or lithium diisopropylamides. The reaction medium is generally constituted by an aprotic solvent, preferably by an aprotic solvent selected from among tetrahydrofuran, dioxane, dimethyl sulfoxide, diglyme and ethyl ether.

As far as we are aware, the 16-phenyl sulfides of the formula (4), in which R' represents hydrogen, methyl or ethyl, and in particular the 16-phenyl sulfides of the formula (4a), in which R' represents ethyl, are new compounds, synthesised for the first time in the process provided by the present invention.

The 16-phenyl sulfide of the formula (4), preferably (4a), obtained in this manner is then subjected, in accordance with step b) of the present process, to an oxidation reaction to yield the 16-phenyl sulfoxide of the formula (5), preferably (5a). Said oxidation reaction from sulfide to sulfoxide is generally performed under conventional conditions, as for example described in J. Am. Chem. Soc. 98, 4887-4902, (1976), preferably using an oxidising agent selected from among m-chloroperbenzoic acid, hydrogen peroxide, sodium metaperiodate, dimethyldioxirane and t-butyl hydroperoxide, still more preferably m-chloroperbenzoic acid. The reaction is generally performed in chlorinated solvents, hydrocarbons, ketones, alcohols, preferably in dichloromethane.

The 16-phenyl sulfoxides of the formula (5), in particular 16ξ-phenyl sulfoxide-18-methyl-3,3-(2',2'-dimethyl-1',3'-propylenedioxy)-5-estren-17-one and 16ξ-phenyl sulfoxide-18-methyl-3,3-(2',2'-dimethyl-1',3'-propylenedioxy)-5(10)-estren-17-one (5a), are not described in the literature and have been prepared for the first time by us.

The 16ξ-phenyl sulfoxide of the formula (5) obtained in this manner takes the form of a complex diastereomeric mixture in that it is constituted by four stereoisomers generated by the presence of the stereogenic centres on the carbon 16 and on the sulfur. This diastereomeric mixture is directly usable in the subsequent elimination reaction (step c)) of the present process to yield compounds of the formula (6), preferably (6a), characterised by the 15-16 double bond.

Said elimination reaction is generally performed in the presence of an organic base such as triethylamine, pyridine or lutidine, preferably triethylamine, in a high-boiling aprotic solvent such as for example xylene, both as a pure isomer and as a mixture of isomers, N,N-dimethylformamide, dimethyl sulfoxide, preferably o-xylene, usually at the reflux temperature of the mixture.

Alternatively, the reaction may be performed under conditions of basic heterogeneous catalysis, for example using xylene as the solvent and basic alumina as the catalyst.

The compounds of the formula (6) obtained in this manner are in turn particularly advantageous intermediates for the preparation of variously substituted Δ¹⁵⁻¹⁶-17-ketosteroids, in particular of gestodene (1).

Indeed, it is possible to convert the intermediate compound of the formula (6), in which R' is ethyl and the two instances of R together represent a divalent group -(CH₂)₂-, -(CH₂)₃-, -CH₂-C(CH₃)₂-CH₂-, preferably the divalent group -CH₂-C(CH₃)₂-CH₂- (6a), into the compound of the formula (7) by means of treatment with a suitable ethynylating agent such ethynylmagnesium bromide (DE2546062) or, preferably, with other ethynylating agents such as acetylene in butyllithium or lithium acetylide complexed with ethylenediamine in tetrahydrofuran. Acid deprotection of the gestodene (7), preferably with 6N hydrochloric acid, accordingly gives rise to gestodene in yields, starting from the intermediate (6a), which are generally around 80% or above.

The physico-chemical properties of the resultant product are in accordance with those stated in the literature for this compound.

One particularly preferred embodiment of the process provided by the present invention, which certainly is surprising in the light of the prior negative tests of bromination and functionalisation with diphenyl diselenide, involves the direct conversion of the compounds of the formula (3), preferably (3a), into compounds of the formula (5), preferably (5a), so considerably simplifying the process and significantly raising overall yields.

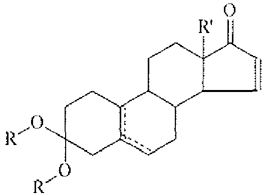

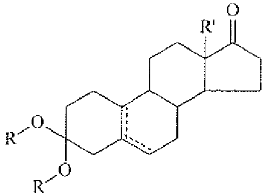

This conversion is conveniently adopted in a preferred embodiment of the present invention, namely in a process for the preparation of gestodene (1) according to the following scheme: 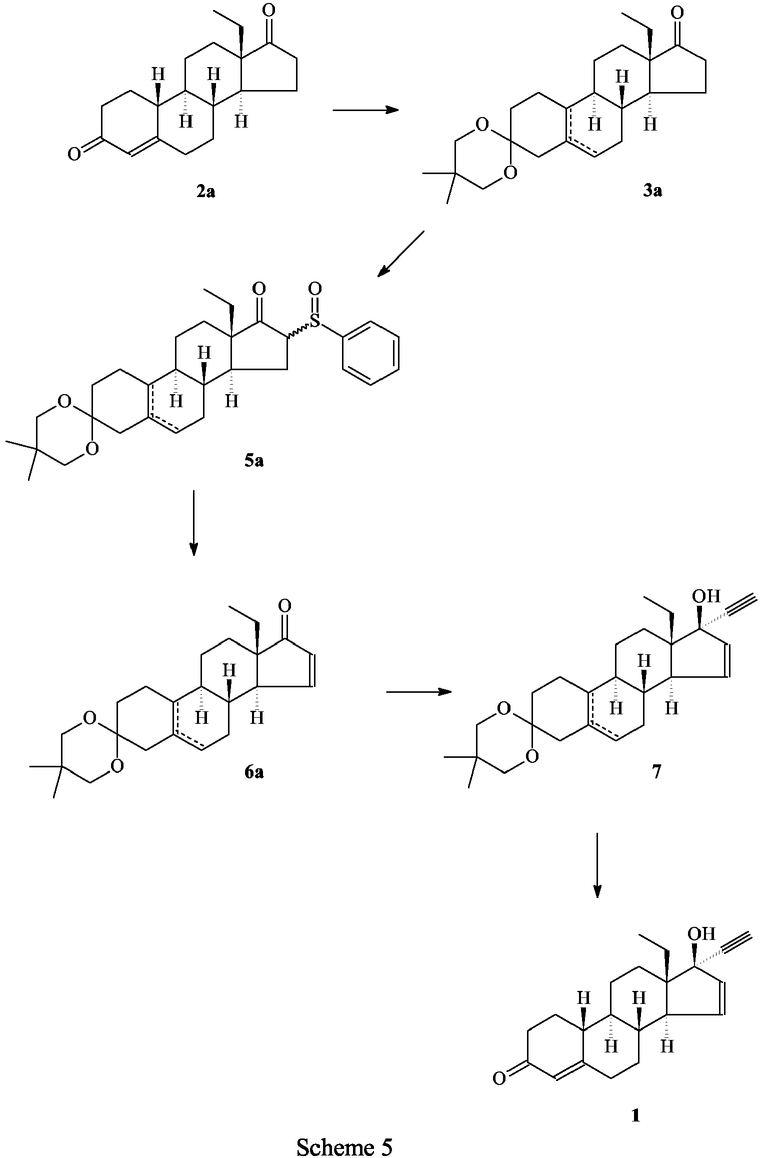 In conclusion, by selecting the ketal as the protective group for the carbon present in position 3 and phenyl sulfoxide as the functional group for position 16 (schemes 4 and 5), we were able to carry out a simple synthesis of Δ¹⁵⁻¹⁶-17-ketosteroids, in particular of gestodene, without using microbiological transformations and with yields comparable to those stated in the literature starting from the same diketones of the formula (2), in particular from 18-methyl-4-estren-3,17-dione (2a).

### Experimental section

### Example 1

### Preparation of 18-methyl-3,3-(2',2'-dimethyl-1',3'-propylenedioxy)-5-estren-17-one and 18-methyl-3,3-(2',2'-dimethyl-1',3'-propylenedioxy)-5(10)-estren-17-one (3a) starting from 18-methyl-4-estren-3,17-dione (2a)

Triethyl orthoformate (1 ml), dimethylpropanediol (1.043 g, 10.03 mmol) and p-toluenesulfonic acid (6 mg) are added to a solution of 18-methyl-4-estren-3,17-dione (2a) (1.049 g, 3.67 mmol) in dichloromethane (11 ml). The reaction mixture is maintained under an inert atmosphere at a temperature of 14°C for 8 hours. A saturated solution of sodium bicarbonate (1-2 ml) is then added. The aqueous phase is separated and the organic phase is washed to neutrality with a saturated solution of sodium chloride. The organic phase is then dried over sodium sulfate and, after filtration, evaporated under reduced pressure. The resultant oily residue (1.35 g) is used without any further purification in the subsequent synthesis step.

The product was purified for analytical purposes by chromatography on silica gel. The ketal (3a) is obtained by elution with 8/2 petroleum ether/ethyl acetate.
¹H-NMR (500 MHz, CDCl₃): δ 0.73 (t, J 7Hz, 0.9H, 18CH₃); 0.75 (t, J 7 Hz, 2.1H, 18-CH₃); 0.85 (s, 0.9H, CH₃-C); 0.89 (s, 2.1H, CH₃-C); 1.00 (s, 2.1H, CH₃-C); 1.06 (s, 0.9H, CH₃-C); 3.44-3.66 (m, 4H, CH₂O); 5.50 (m, 0.7H, CH=) IR (1% KBr)= 3449.5 cm⁻¹, 2952.2 cm⁻¹, 2866.7 cm⁻¹, 1732.2 cm⁻¹ (C=O st).
Calculated elemental analysis for C₂₄H₃₆O₃: theory: C=77.38%; H=9.74%; O=12.88%; actual: C=77.45%; H=9.69%; O=12.95%
Melting point: 90-110°C

### Example 2

### Preparation of 16ξ-phenyl sulfoxide-18-methyl-3,3-(2',2'-dimethyl-1',3'-propylenedioxy)-5-estren-17-one and 16ξ-phenyl sulfoxide-18-methyl-3,3-(2',2'-dimethyl-1',3'-propylenedioxy)-5(10)-estren-17-one (5a) from 18-methyl-3,3-(2',2'-dimethyl-1',3'-propylenedioxy)-5-estren-17-one and 18-methyl-3,3-(2',2'-dimethyl-1',3'-propylenedioxy)-5(10)-estren-17-one (3a)

Potassium *tert*.-butylate (0.79 g, 7.06 mmol) was added under an inert atmosphere with vigorous stirring to a solution of compound (3a) (0.65 g, 1.73 mmol) in anhydrous tetrahydrofuran (14.2 ml). The reaction mixture was stirred at ambient temperature for 10', then methyl benzenesulfinate (0.91 ml, d 1.194, 6.96 mmol) was added and the reaction composition was stirred for 2 hours at ambient temperature. After this period, ethyl acetate (20 ml) is added and the organic phase is washed successively with a solution of sodium chloride (3x30 ml), water (30 ml) and then with a saturated sodium chloride solution (30 ml). The organic phase is dehydrated over sodium sulfate, filtered and concentrated down to a residue under reduced pressure. The resultant oily product (5a) (0.850 g; 1.71 mmol) is used in the subsequent step without purification.
Mass fragmentation analysis (m/z): 498 (m+1); 497 (m+/.); 371 (m-126); 370 (m-127)
IR (1% KBr)= 3449.9 cm⁻¹, 2954.9 cm⁻¹, 2868.9 cm⁻¹, 1735.9 cm⁻¹ (C=O st); 1129.6, 1106.5, 1093.8 and 1048.7 cm⁻¹ (S=O st).

### Example 3

### Preparation of 16ξ-phehyl sulfide-18-methyl-3,3-(2',2'-dimethyl-1',3'-propylenedioxy)-5-estren-17-one and 16ξ-phenyl sulfide-18-methyl-3,3-(2',2'-dimethyl-1',3'-propylenedioxy)-5(10)-estren-17-one (4a) from 18-methyl-3,3-(2',2'-dimethyl-1',3'-propylenedioxy)-5-estren-17-one and 18-methyl-3,3-(2',2'-dimethyl-1',3'-propylenedioxy)-5(10)-estren-17-one (3a)

A solution of diisopropylamine (0.42 ml) in anhydrous tetrahydrofuran (3.6 ml) is added with stirring at -78°C under an inert atmosphere to a 1.6 M solution of n-butyllithium in hexane (1.9 ml; 3.04 mmol). The reaction mixture is stirred at -78°C for 20 minutes and then a solution of 18-methyl-3,3-(2',2'-dimethyl-1',3'-propylenedioxy)-5-estren-17-one and of 18-methyl-3,3-(2',2'-dimethyl-1',3'-propylenedioxy)-5(10)-estren-17-one (3a) (558 mg;1.5 mmol) in 3.6 ml of anhydrous tetrahydrofuran is added in succession. Stirring is continued for a further 40', then diphenyl disulfide (393 mg; 1.8 mmol) and 1.2 ml of hexamethylphosphorotriamide (HMPTA) are added in succession. The reaction mixture is adjusted to ambient temperature and stirred for 3 hours and then poured into 50 ml of a mixture consisting of ethyl acetate and 10% HCl (1/1 vol./vol.). The organic phase is separated, washed with a saturated bicarbonate solution (15 ml), dehydrated over sodium sulfate, filtered and concentrated under a vacuum to yield an oily residue which is purified by chromatography on silica gel (1/20 wt./wt.): The 16ξ-phenyl sulfide-18-methyl-3,3-(2',2'-dimethyl-1',3'-propylenedioxy)-5 -estren-17-one and 16ξ-phenyl sulfide-18-methyl-3,3-(2',2'-dimethyl-1',3'-propylenedioxy)-5(10)-estren-17-one (4a) is recovered as an oil (432 mg, 0.9 mmol; 60% yield) by elution with 7/3 hexane/ethyl acetate.
Mass fragmentation analysis (m/z): 482 (m+1); 481 (m+/.); 372 (m-109); 370 (m-127)

### Example 4

### Preparation of 16ξ-phenyl sulfoxide-18-methyl-3,3-(2',2'-dimethyl-1',3'-propylenedioxy)-5-estren-17-one and 16ξ-phenyl sulfoxide-18-methyl-3,3-(2',2'-dimethyl-1',3'-propylenedioxy)-5(10)-estren-17-one (5a) from 16ξ-phenyl sulfide-18-methyl-3,3-(2',2'-dimethyl-1',3'-propylenedioxy)0-5-estren-17-one and 16ξ-phenyl sulfide-18-methyl-3,3-(2',2'-dimethyl-1',3'-propylenedioxy)-5(10)-estren-17-one (4a)

The 16ξ-phenyl sulfide-18-methyl-3,3-(2',2'-dimethyl-1',3'-propylenedioxy)-5 -estren-17-one and 16ξ-phenyl sulfide-18-methyl-3,3-(2',2'-dimethyl-1',3'-propylenedioxy)-5(10)-estren-17-one (274 mg; 0.57 mmol) (4a) are dissolved with stirring at ambient temperature in anhydrous dichloromethane (13.5 ml); the reaction mixture is cooled to -70°C and, in 15', a solution of m-chloroperbenzoic acid (113 mg) in dichloromethane (5 ml) is added.

The reaction mixture is stirred at -70°C for 4 hours; it is poured with stirring at ambient temperature into a mixture consisting of ethyl ether (50 ml) and 10% aqueous sodium sulfite (50 ml). The organic phase is then washed with sodium bicarbonate (40 ml), with water, is dehydrated over sodium sulfate, filtered and concentrated to yield a residue which is purified by chromatography on silica gel (1/20 wt./wt.): The 16ξ-phenyl sulfoxide-18-methyl-3,3-(2',2'-dimethyl-1',3'-propylenedioxy)-5-estren-17-one and 16ξ-pheny sulfoxide-18-methyl-3,3-(2',2'-dimethyl-1',3'-propylenedioxy)-5(10)-estren-17-one (84 mg, 0,17 mmol; 30% yield) (5a), the physico-chemical properties of which are similar to those stated in Example 2, is recovered by elution with 1/1 hexane/ethyl acetate.

### Example 5

### Preparation of 18-methyl-3,3-(2',2'-dimethyl-1',3'-propylenedioxy)-5,15-estradien-17-one and 18-methyl-3,2-(2',2'-dimethyl-1',3'-propylenedioxy)-5(10),15-estradien-17-one (6a) from 16ξ-pheny, sulfoxide-18-methyl-3,3-(2',2'-dimethyl-1',3'-propylenedioxy)-5-estren-17-one and 16ξ-phenyl sulfoxide-18-methyl-3,3-(2',2'-dimethyl-1',3'-propylenedioxy)-5(10)-estren-17-one (5a)

Triethylamine (0.5 ml) is added with stirring under an inert atmosphere to a solution of compound (5a) (0,5 g, 1,0 mmol) in o-xylene (10 ml). The reaction mixture is refluxed (138°C) under an inert atmosphere for 2.5 hours. After this period, toluene (20 ml) is added to the reaction mixture which has cooled to ambient temperature, then the organic phase is washed with water (3 x 20 ml) and with a saturated aqueous sodium chloride solution (20 ml). The organic phase is then dehydrated over sodium sulfate, filtered and concentrated down to a residue under reduced pressure. The crude product (0.48 g) is purified by chromatography on silica (12 g): 0.23 g of compound (5a) (61% from compound 3a by the pathway of Example 2) are obtained by elution with 9/1 petroleum ether/ethyl acetate.
¹H-NMR (500 MHz, CDCl₃) d: 0.74 (t, J=7 Hz, 1.2H, CH₃-18); 0.77 (t, J=7 Hz, 1.8H, CH₃-18); 0.86 (s, 1.2H, CH₃-C); 0.90 (s, 1.8H, CH₃-C); 1.01 (s, 1.8H, CH₃-C); 1.08 (s, 1.2H, CH₃-C); 3.40-3.70 (m, 4H, CH₂O); 5.50 (m, 0.4H, CH=); 5.99 (dd, J=2.8 and 5.6 Hz, 1H, CH=); 7.47 (D, J=5.6 Hz, 0.6H, CH=); 7.54 (D, J=5.6 Hz, 0.4H, CH=).
Mass fragmentation analysis (m/z): 372 (m+1); 371 (m+/.); 341 (m-30).
IR (1% KBr)= 3454.0 cm⁻¹, 2951.3 cm⁻¹, 2863.7 cm⁻¹, 1706.0 cm⁻¹ (C=O st).
Calculated elemental analysis for C₂₄H₃₄O₃: theory: C=77.80%; H=9.25%; O=12.95%; actual: C=77.65%; H=9.19%; O=12.84%
Melting point: 115-125°C

### Example 6

### Preparation of 17α-ethynyl-18-methyl-3,3-(2',2'-dimethyl-1',3'-propylenedioxy)-5,15-estradien-17β-ol and 17α-ethynyl-18-methyl-3,3-(2',2'-dimethyl-1',3'-propylenedioxy)-5(10),15-estradien-17β-ol (7) from 18-methyl-3,3-(2',2'-dimethyl-1',3'-propylenedioxy)-5,15-estradien-17-one and 18-methyl-3,3-(2',2'-dimethyl-1',3'-propylenedioxy)-5(10),15-estradien-17-one (6a)

Lithium acetylide complexed with ethylenediamine (0.18 g, 1.98 mmol) is added at 0°C under nitrogen to a solution of compound (6a) (0.16 g, 0.43 mmol) in anhydrous tetrahydrofuran (4 ml). The reaction is performed at 0°C under nitrogen for 1 hour. After this period, a 1N solution of hydrochloric acid is added until pH 6 is reached (3.5 ml) and the reaction mixture is extracted with ethyl acetate (15 ml). The organic phase is washed with water (3x15 ml). The organic phase is dried over sodium sulfate, filtered and the solvents are evaporated under reduced pressure. Compound (7) (0.16 g, 94% yield) is obtained.
¹H-NMR (500 MHz, CDCl₃)δ: 0.82 (t, J=7 Hz, 1.35H, CH₃-CH₂); 0.85 (s, 1.35H, CH₃-CH₂); 0.86 (t, J=7 Hz, 1.65H, CH₃-C); 0.90 (s, 1.65H, CH₃-C); 1.00 (s, 1.65H, CH₃-C); 1.06 (s, 1.35H, CH₃-C); 2.60 (s, 1.35H, CH-21); 2.61 (s, 1.65H, CH-21); 3.40-3.70 (m, 4H, CH₂O); 5.46 (m, 0.55H, CH=); 5.67 (dd, J=3.5 and 5.6 Hz, 1H, CH=); 5.90 (dd, J=1.7 and 5.6 Hz, 0.55H, CH=); 5.99 (dd, J=1.7 and 5.6 Hz, 0.45H, CH=).
Mass fragmentation analysis (m/z): 398 (m+1); 397 (m+/.); 380 (m-17); 355 (m-42).
Calculated elemental analysis for C₂₆H₃₆O₃: theory: C=78.75%; H=9.15%; O=12.10%; actual: C=78.63%; H=9.10%; O=11.98 %
DSC (5°C/min) = presence of two fusion endotherms with peaks at 174 and 192°C.

### Example 7

### Preparation of gestodene (1) from 17α-ethynyl-18-methyl-3,3-(2',2'-dimethyl-1',3'-propylenedioxy)-5,15-estradien-17β-ol and 17α-ethynyl-18-methyl-3,3-(2',2'-dimethyl-1',3'-propylenedioxy)-5(10),15-estradien-17β-ol (7)

A 6N solution of hydrochloric acid (0.1 ml) is added to a solution of compound (7) (0.16g, 0.404 mmol) in acetone (3 ml). The reaction mixture is stirred at ambient temperature for 45 minutes, then a saturated solution of sodium hydrogencarbonate is added until a neutral pH is obtained and the organic solvent is evaporated under reduced pressure. The mixture obtained is extracted with ethyl acetate (2 x 2 ml) and the organic phase is dried over sodium sulfate, filtered and concentrated down to a residue under reduced pressure. The resultant residue (0.12 g) is purified by chromatography on silica gel (1 g): 0.113 g of gestodene (0.36 mmol; 90% yield) are recovered by elution with 9/1 petroleum ether/ethyl acetate.
¹H-NMR (500 MHz, CDCl₃)δ: 0.90 (t, J=7 Hz, 3H, CH₃-CH₂); 2.62 (s, 1H, H-21); 3.40-3.70 (m, 4H, CH₂O); 5.71 (dd, 1H, CH=16); 5.84 (m, 1 H, CH=4); 5.93 (dd, 1 H, CH=15)
DSC (5°C/min) = peak at 199.75°C (onset at 198.90°C)
IR (1% KBr)= 3313.7 cm⁻¹ (OH st), 3233.4 cm⁻¹ (=CH st), 1699.9 cm⁻¹ (C=O).
Mass fragmentation analysis (m/z): 312 (m+2); 311 (m+1); 310 (m+/.); 293 (m-17).
[α]_{D}=-187.8 (c=1 CH₂Cl₂)
Calculated elemental analysis for C₂₁H₂₆O₂: theory: C=81.21%; H=8.44%; O=10.31%; actual C=80.99%, H=8.47%; O=10.50 %.

## Claims

1. A process for the preparation of Δ¹⁵⁻¹⁶-17-ketosteroids of the formula 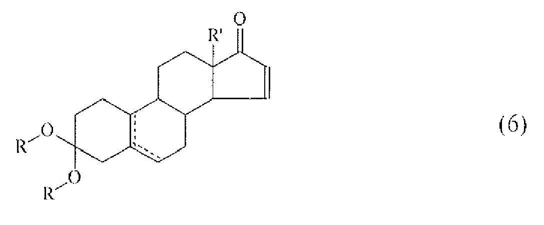 in which
R' represents hydrogen, methyl or ethyl,
the two groups R together represent a divalent group -(CH₂)₂-, -(CH₂)₃-, - CH₂-C(CH₃)₂-CH₂-
and the dashed line represents a 5-6 or 5-10 double bond, or a mixture thereof
which comprises:
a) the reaction of a 17-ketosteroid of the formula 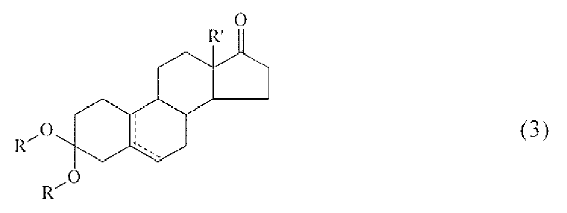 in which R, R' and the dashed line have the above-stated meanings, with diphenyl disulfide in the presence of a strong base to yield the compound of the formula 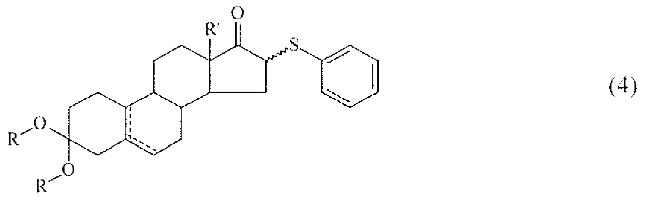 in which R, R' and the dashed line have the above-stated meanings,
b) the subsequent oxidation reaction of the compound of the formula (4) to yield the compound of the formula 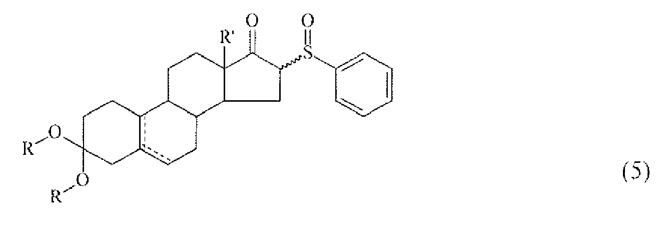 in which R, R' and the dashed line have the above-stated meanings, and
c) the elimination reaction of the compound of the formula (5) to yield the Δ¹⁵⁻¹⁶-17-ketosteroid compound of the formula (6), wherein said elimination reaction is performed in the presence of an organic base in a high-boiling aprotic solvent selected from among xylene, N,N-dimethylformamide, dimethyl sulfoxide or is performed under conditions of basic heterogeneous catalysis.

2. The process according to claim 1 in which the two groups R together represent -CH2-C(CH₃)₂-CH₂- , R' is ethyl and the dashed line has the above-stated meaning.

3. The process according to claim 1 or claim 2 which further comprises preparation of the ketal of the formula 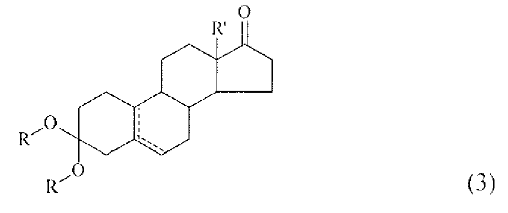 in which R, R' and the dashed line have the above-mentioned meanings, by selective ketalisation in position 3 of the corresponding 3,17-diketosteroid of the formula 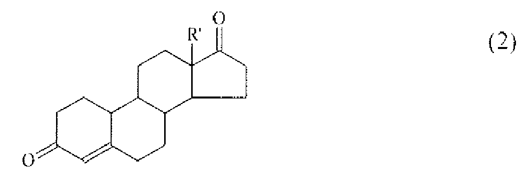 with a diol of the formula HO-(CH₂)₂-OH, HO-(CH₂)₃-OH, HO-CH₂-C(CH₃)₂-CH₂-OH, in the presence of a suitable acid catalyst.

4. The process according to claim 3, in which said ketal of the formula 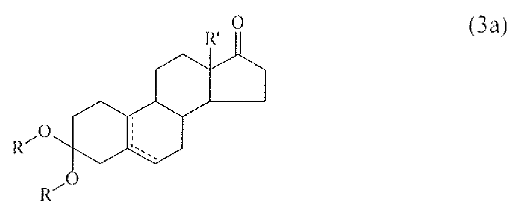 in which the two groups R together represent -CH₂-C(CH₃)₂-CH₂- and R' is ethyl, is prepared by selective ketalisation in position 3 of the corresponding 3,17-diketosteroid of the formula 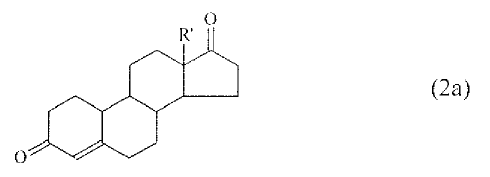 in which R' is ethyl,
said ketalisation being performed with 2,2-dimethylpropanediol, triethyl orthoformate in the presence of p-toluenesulfonic acid.

5. The process according to any one of claims 1 to 4 in which:
- step a) is performed in the presence of a strong base selected from among potassium t-butylate, sodium t-butylate, sodium hydride, n-butyllithium and lithium diisopropylamides, in an aprotic solvent selected from among tetrahydrofuran, dioxane, dimethyl sulfoxide, diglyme and ethyl ether;
- step b) is performed with an oxidising agent selected from among m-chloroperbenzoic acid, hydrogen peroxide, sodium metaperiodate, dimethyldioxirane and t-butyl hydroperoxide;
- step c) is performed in the presence of an organic base selected from among triethylamine, pyridine and lutidine, in a high-boiling aprotic solvent selected from among xylene, N,N-dimethylformamide, dimethyl sulfoxide, preferably with triethylamine in o-xylene.

6. A process for the preparation of gestodene of the formula 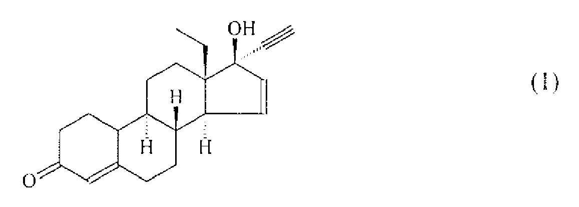 which comprises one of the processes according to any one of claims 1 to 5.

7. The process for the preparation of gestodene according to claim 6 which further comprises:
- the ethynylation reaction of the compound of the formula 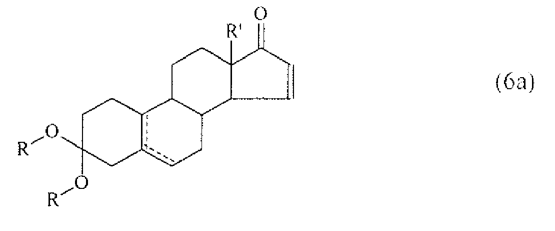 in which the two groups R together represent -CH₂-C(CH₃)₂-CH₂- and R' is ethyl,
to yield the intermediate of the formula 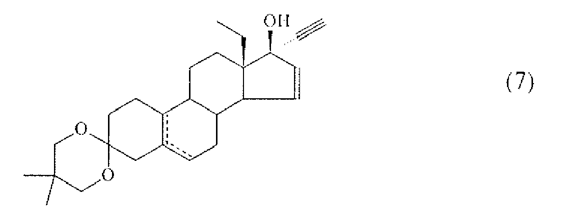 and
- subsequent acid deprotection to yield the gestodene (1).

8. A compound of the formula 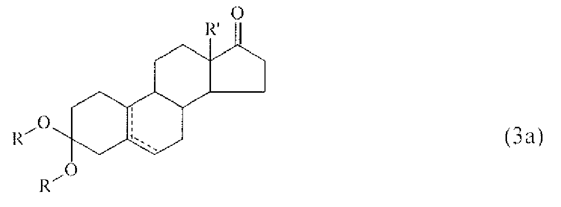 in which
R' represents ethyl,
the two groups R together represent -CH₂-C(CH₃)₂CH₂- , and
the dashed line represents a 5-6 or 5-10 double bond.

9. A compound of the formula 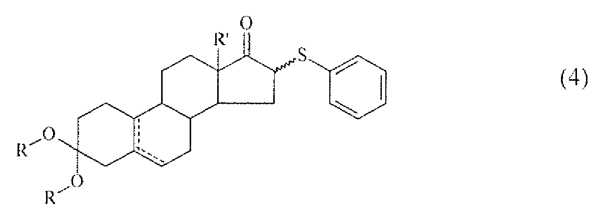 in which
R' represents hydrogen, methyl or ethyl, and the two groups R together represent a divalent group -(CH₂)₂-, -(CH₂)₃-, -CH₂-C(CH₃)₂-CH₂-, and the dashed line represents a 5-6 or 5-10 double bond.

10. The compound according to claim 9 in which R' represents ethyl, and the two groups R together represent -CH₂-C(CH₃)₂-CH₂-.

11. A compound of the formula 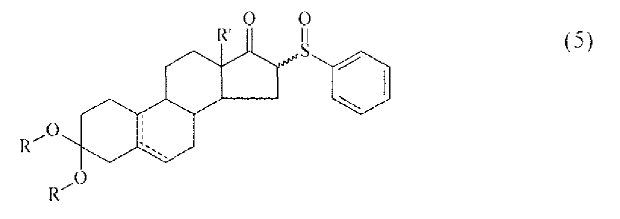 in which
R' represents hydrogen, methyl and ethyl,
the two instances of R together represent a divalent group -(CH₂)₂-, - (CH₂)₃-, -CH₂-C(CH₃)₂-CH₂-, and
the dashed line represents a 5-6 or 5-10 double bond.

12. The compound according to claim 11 in which R' represents ethyl and the two groups R together represent -CH₂-C(CH₃)₂-CH₂-.

13. Use of the compounds of the formula (3), (4) or (5) for the preparation of
Δ¹⁵⁻¹⁶-17-ketosteroids of the formula 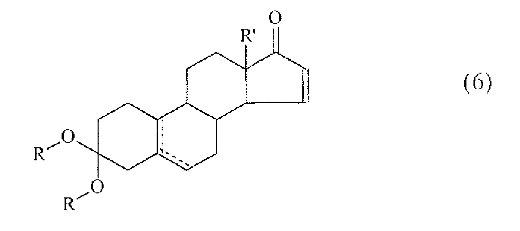 in which R' represents hydrogen, methyl or ethyl,
the two groups R together represent a divalent group -(CH₂)₂-, -(CH₂)₃-, - CH₂-C(CH₃)₂-CH₂-
and the dashed line represents a 5-6 or 5-10 double bond.

14. Use of the compounds of the formula (3a), 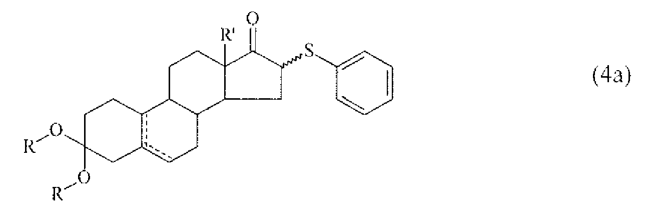 or 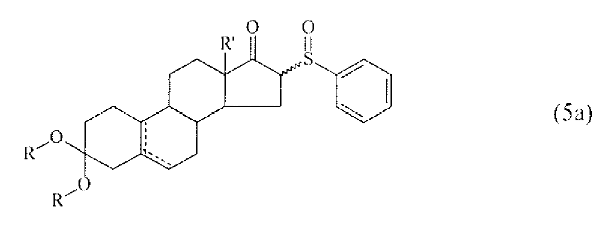 for the preparation of
a compound of the formula 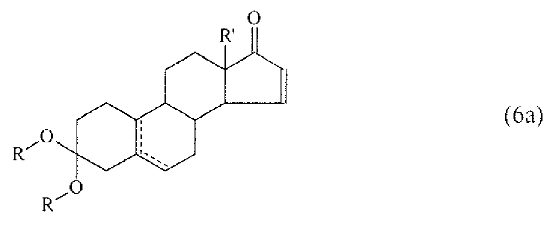 in which the two groups R together represent -CH₂-C(CH₃)₂-CH₂-, the dashed line represents a 5-6 or 5-10 double bond and R' is ethyl.

15. Use of the compounds of the formula (3a), 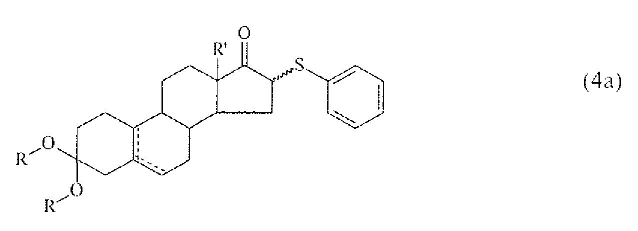 or 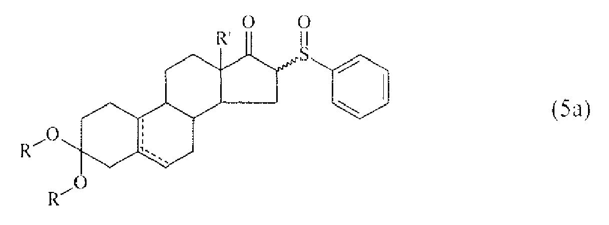 in which the two groups R together represent -CH₂-C(CH₃)₂-CH₂-, the dashed line represents a 5-6 or 5-10 double bond and R' is ethyl for the preparation of gestodene (1).

## Patentansprüche

1. Verfahren zur Herstellung von Δ¹⁵⁻¹⁶-17-Ketodteroiden der Formel 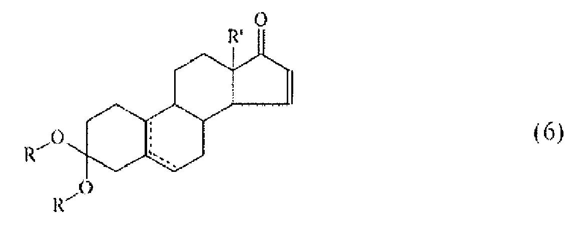 worin
R' Wasserstoff, Methyl oder Ethyl darstellt,
die zwei R-Gruppen zusammen eine zweiwertige Gruppe -(CH₂)₂-, -(CH₂)₃-, -CH₂-C(CH₃)₂-CH₂- darstellen,
und die gestrichelte Linie eine 5-6 oder 5-10 Doppelbindung darstellt, oder ein Gemisch davon,
welches umfasst:
a) die Reaktion eines 17-Ketosteroids der Formel 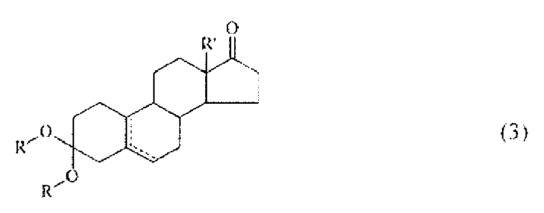 worin R, R' und die gestrichelten Linie wie oben definiert sind, mit Diphenylsulfid in Anwesenheit einer starken Base,
um die Verbindung der Formel 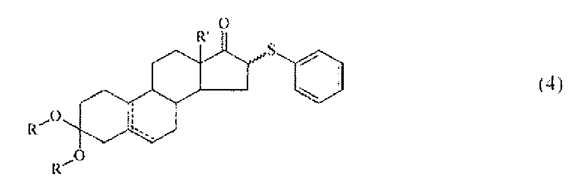 zu erhalten
worin R, R' und die gestrichelten Linie wie oben definiert sind,
b) die nachfolgende Oxidationsreaktion der Verbindung der Formel (4), um die Verbindung der Formel 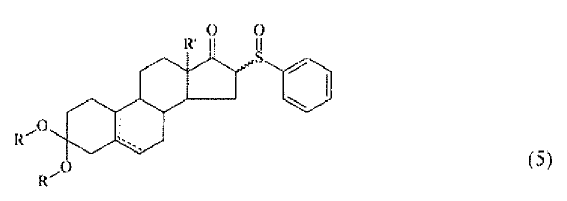 zu erhalten, worin R, R' und die gestrichelten Linie wie oben definiert sind, und
c) die Eliminierungsreaktion der Verbindung der Formel (5), um die Δ¹⁵⁻¹⁶-17-Ketosteroid-Verbindung der Formel (6) zu erhalten, worin die Eliminierungsreaktion in Anwesenheit einer organischen Base in einem hochsiedenden aprotischen Lösungsmittel durchgerührt wird, ausgewählt unter Xylol, N,N-Dimethylformamid, Dimethylsulfoxid, oder unter Bedingungen einer basischen heterogenen Katalyse durchgeführt wird.

2. Verfahren nach Anspruch 1, wobei die zwei R-Gruppen zusammen -CH₂-C(CH₃)₂-CH₂- darstellen, R' Ethyl und die gestrichelte Linie wie oben definiert ist.

3. Verfahren nach Anspruch 1 oder 2, welches weiter die Zubereitung des Ketals der Formel 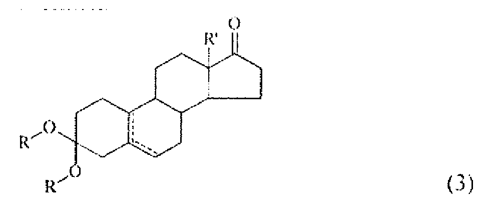 umfasst,
worin R, R' und die gestrichelte Linie wie oben definiert sind,
durch selektive Ketalisierung an Position 3 des entsprechenden 3,17-Diketosteroids der Formel 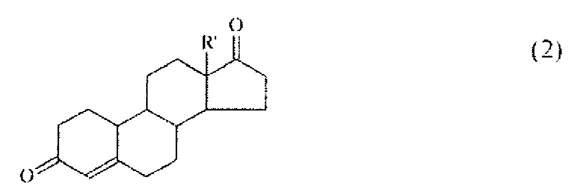 mit einem Diol der Formel HO-(CH₂)₂-OH, HO-(CH₂)₃-OH, HO-CH₂-C(CH₃)₂-CH₂-OH in Anwesenheit eines geeigneten Säure-Katalsysators.

4. Verfahren nach Anspruch 3, wobei das Ketal der Formel 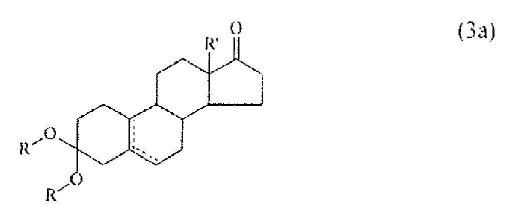 wobei die zwei R-Gruppen zusammen -CH₂-C (CH₃)₂-CH₂- darstellen und R' Ethyl ist,
durch selektive Ketalisierung an Position 3 des entsprechenden 3,17- Diketosteroids der Formel 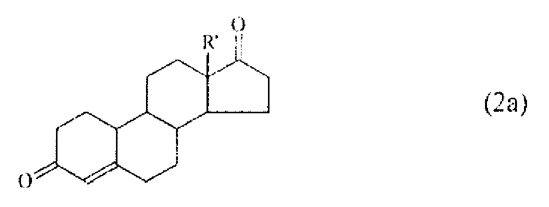 worin R' Ethyl ist,
hergestellt wird
worin die Ketalisierung mit 2,2-Dimethylpropandiol, Triethylorthoformat in Anwesenheit von p-Toluolsulfonsäure durchgeführt wird.

5. Verfahren nach einem der Ansprüche 1 bis 4, wobei
- Schritt a) durchgeführt wird in Anwesenheit einer starken Base ausgewählt unter Kalium-t-Butylat, Natrium-t-Butylat, Natriumhydrid, n-Butyllithium und Lithiumdiisopropylamiden, in einem aprotischen Lösungsmittel ausgewählt unter Tetrahydrofuran, Dioxan, Dimethylsulfoxid, Diglyme und Ethylether;
- Schritt b) durchgeführt wird mit einem Oxidierungsmittel ausgewählt unter m-Chlorbenzoesäure, Wasserstoffperoxid, Natriummetaperiodat, Dimethyldioxiran und t-Butyl-Wasserstoffperoxid;
- Schritt c) durchgeführt wird in Anwesenheit einer organischen Base ausgewählt unter Triethylamin, Pyridin und Lutidin, in einem hoch-siedenden aprotischen Lösungsmittel ausgewählt unter Xylol, N,N-Dimethylformamid, Dimethylsulfoxid, vorzugsweise mit Triethylamin in o-Xylol.

6. Verfahren zur Herstellung von Gestoden der Formel 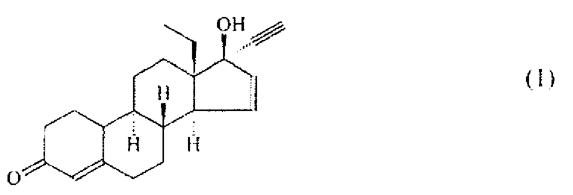 welches eines der Verfahren nach einem der Ansprüche 1 bis 5 umfasst.

7. Verfahren zur Herstellung von Gestoden nach Anspruch 6, welches weiter umfasst:
- die Ethinylisierungsreaktion der Verbindung der Formel 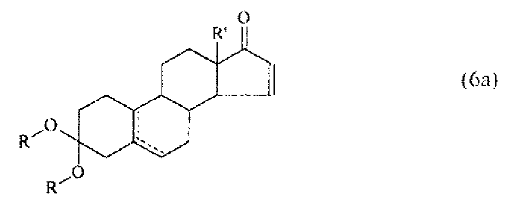 worin die zwei R-Gruppen zusammen -CH₂-C(CH₃)₂-CH₂-, darstellen und R' Ethyl ist,
um die Zwischenverbindung der Formel 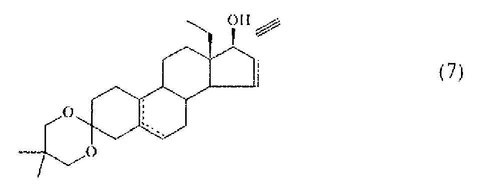 zu erhalten, und
- nachfolgende Entfernung der Schutzgruppe durch Säure, um das Gestoden (1) zu erhalten.

8. Verbindung der Formel 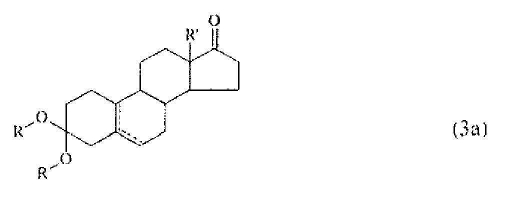 worin R' Ethyl ist,
die zwei R-Gruppen zusammen -CH₂-C(CH₃)₂-CH₂- darstellen, und die gestrichelte Linie eine 5-6 oder 5-10 Doppelbindung darstellt.

9. Verbindung der Formel 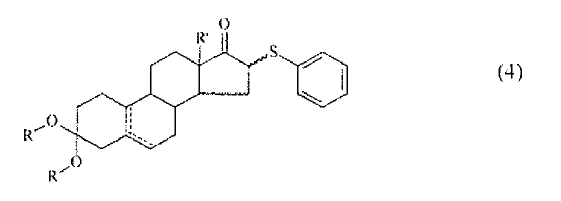 worin
R' Wasserstoff, Methyl oder Ethyl darstellt,
die zwei R-Gruppen zusammen eine zweiwertige Gruppe -{CH₂)₂-, -(CH₂)₃-, -CH₂-C(CH₃)₂-CH₂- darstellen,
und die gestrichelte Linie eine 5-6 oder 5-10 Doppelbindung darstellt

10. Verbindung nach Anspruch 9, worin R' Ethyl darstellt und die zwei R-Gruppen zusammen -CH₂-C(CH₃)₂-CH₂- darstellen.

11. Verbindung der Formel 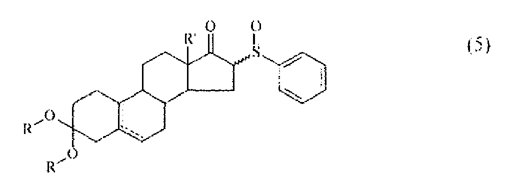 worin
R' Wasserstoff, Methyl oder Ethyl darstellt,
die zwei Möglichkeiten von R zusammen eine zweiwertige Gruppe (CH₂)₂-, -(CH₂)₃-, -CH₂-C(CH₃)₂-CH₂- darstellen,
und die gestrichelte Linie eine 5-6 oder 5-10 Doppelbindung darstellt.

12. Verbindung nach Anspruch 11, worin R' Ethyl und die zwei R-Gruppen zusammen -CH₂-C(CH₃)₂-CH₂- darstellen.

13. Verwendung der Verbindungen der Formel (3), (4) und (5) zur Herstellung von Δ¹⁵⁻¹⁶-17-Ketosteroiden der Formel 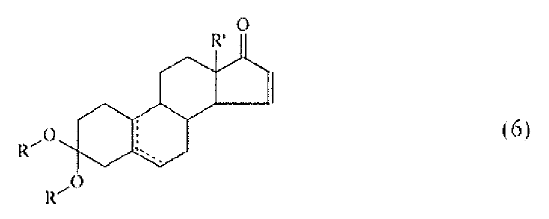 worin
R' Wasserstoff, Methyl oder Ethyl darstellt,
die zwei R-Gruppen zusammen eine zweiwertige Gruppe -(CH₂)₂-, -(CH₂)₃-, -CH₂-C(CH₃)₂-CH₂- darstellen,
und die gestrichelte Linie eine 5-6 oder 5-10 Doppelbindung darstellt.

14. Verwendung der Verbindungen der Formel (3a) 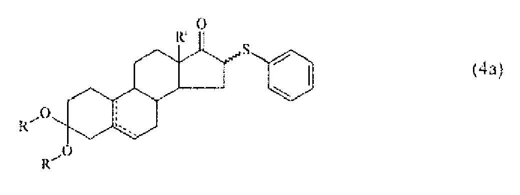 oder 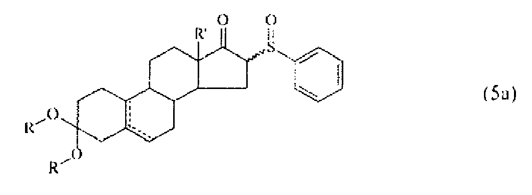 zur Herstellung einer Verbindung der Formel 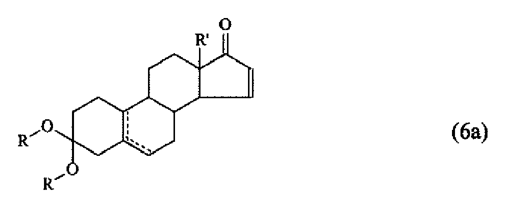 worin
die zwei R-Gruppen zusammen -CH₂-C(CH₃)₂-CH₂- darstellen, die gestrichelte Linie eine 5-6 oder 5-10 Doppelbindung und R' Ethyl ist.

15. Verwendung der Verbindungen der Formel (3a) oder 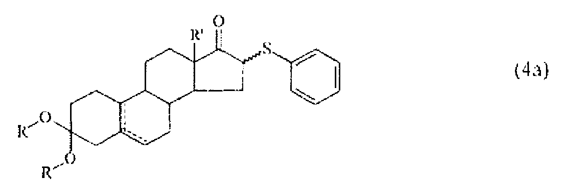 oder 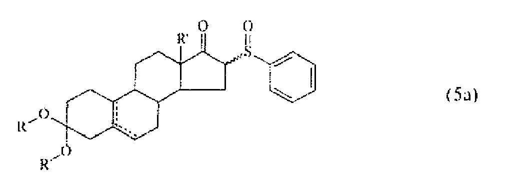 worin die zwei R-Gruppen zusammen -CH₂-C(CH₃)₂-CH₂- darstellen, die gestrichelte Linie eine 5-6 oder 5-10 Doppelbindung und R' Ethyl ist, zur Herstellung von Gestoden (1).

## Revendications

1. Procédé de préparation de Δ^{15,16}-17-cétostéroïdes de formule 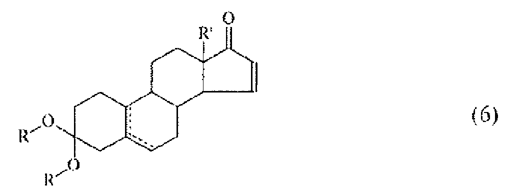 dans laquelle
R' représente un hydrogène, un méthyle ou un éthyle,
les deux groupes R représentent un même groupe divalent -(CH₂)₂-, -(CH₂)₃-,-CH₂-C(CH₃)₂-CH₂-,
et la ligne en pointillé représente une double liaison 5-6 ou 5-10, ou leur mélange qui comprend :
a) la réaction d'un 17-cétostéroïde de formule 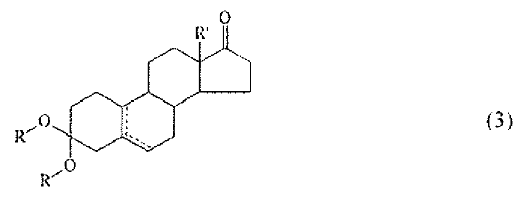 dans laquelle R, R' et la ligne en pointillé ont les significations mentionnées ci-dessus,
avec du disulfure de diphényle en présence d'une base forte
pour donner le composé de formule 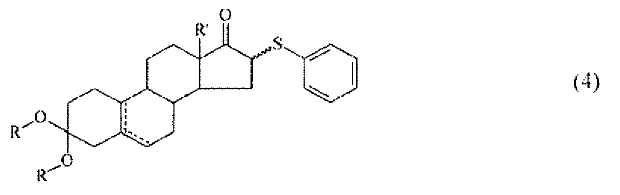 dans laquelle R, R' et la ligne en pointillé ont les significations mentionnées ci-dessus,
b) la réaction d'oxydation ultérieure du composé de formule (4) pour donner le composé de formule 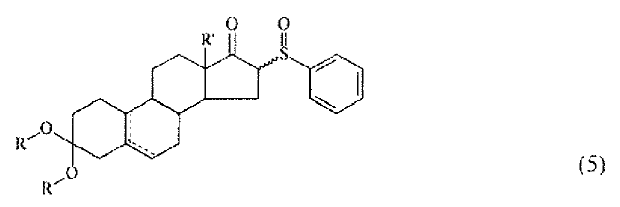 dans laquelle R, R' et la ligne en pointillé ont les significations mentionnées ci-dessus, et
c) la réaction d'élimination du composé de formule (5) pour donner le composé Δ^{15,16}-17-cétostéroïde de formule (6), dans laquelle ladite réaction d'élimination est réalisée en présence d'une base organique dans un solvant aprotique de point d'ébullition élevé choisi parmi le xylène, le N,N-diméthylformamide, le diméthylsulfoxyde ou est réalisée dans des conditions de catalyse hétérogène basique.

2. Procédé selon la revendication 1, dans lequel les deux groupes R représentent tous deux -CH₂-C(CH₃)₂-CH₂-, R' est un éthyle et la ligne en pointillé a la signification mentionnée ci-dessus.

3. Procédé selon la revendication 1 ou la revendication 2, qui comprend en outre la préparation du cétal de formule 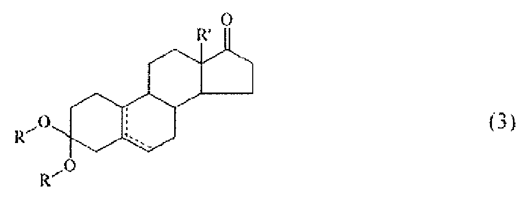 dans laquelle R, R' et la ligne en pointillé ont les significations mentionnées ci-dessus,
par cétalisation sélective en position 3 du 3,17-dicétostéroïde correspondant de formule 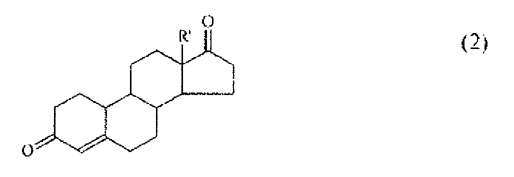 avec un diol de formule HO-(CH₂)₂-OH, HO-(CH₂)₃-OH, HO-CH₂-C(CH₃)₂-CH₂-OH, en présence d'un catalyseur acide approprié.

4. Procédé selon la revendication 3, dans lequel ledit cétal de formule 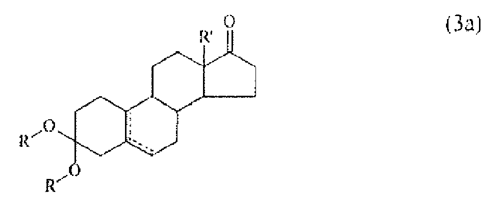 dans laquelle les deux groupes R représentent tous deux -CH₂-C(CH₃)₂-CH₂- et R' est un éthyle, est préparé par cétalisation sélective en position 3 du 3,17-dicétostéroïde correspondant de formule 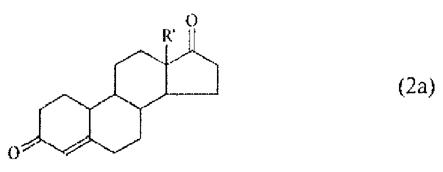 dans laquelle R' est un éthyle,
ladite cétalisation étant réalisée avec du 2,2-diméthylpropanediol, de l'orthoformiate de triéthyle, en présence d'acide p-toluènesulfonique.

5. Procédé selon l'une quelconque des revendications 1 à 4, dans lequel :
- l'étape a) est réalisée en présence d'une base forte choisie parmi le t-butylate de potassium, le t-butylate de sodium, l'hydrure de sodium, les diisopropylamides de n-butyllithium et de lithium, dans un solvant aprotique choisi parmi le tétrahydrofurane, le dioxane, le diméthylsulfoxyde, le diglyme et l'éther éthylique ;
- l'étape b) est réalisée avec un agent d'oxydation choisi parmi l'acide m-chloroperbenzoïque, le peroxyde d'hydrogène, le métapériodate de sodium, le diméthyldioxirane et l'hydroperoxyde de t-butyle ;
- l'étape c) est réalisée en présence d'une base organique choisie parmi la triéthylamine, la pyridine et la lutidine, dans un solvant aprotique de point d'ébullition élevé choisi parmi le xylène, le N,N-diméthylformamide, le diméthylsulfoxyde, de préférence avec de la triéthylamine dans de l'o-xylène.

6. Procédé de préparation de gestodène de formule 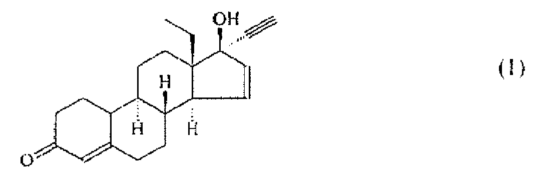 qui comprend un des procédés selon l'une quelconque des revendications 1 à 5.

7. Procédé de préparation de gestodène selon la revendication 6, qui comprend en outre:
- la réaction d'éthynylation du composé de formule 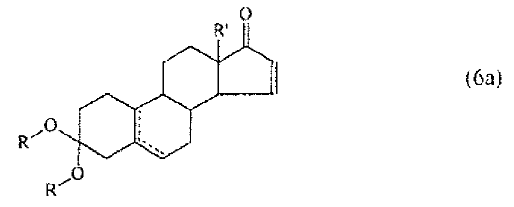 dans laquelle les deux groupes R représentent tous deux CH₂-C(CH₃)₂-CH₂- et R' est un éthyle,
pour donner l'intermédiaire de formule 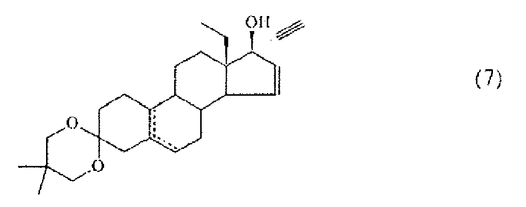 et
- la déprotection acide ultérieure pour donner le gestodène (1).

8. Composé de formule 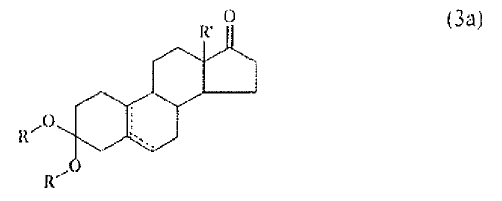 dans laquelle
R' représente un éthyle,
les deux groupes R représentent tous deux -CH₂-C(CH₃)₂-CH₂-, et
et la ligne en pointillé représente une double liaison 5-6 ou 5-10.

9. Composé de formule 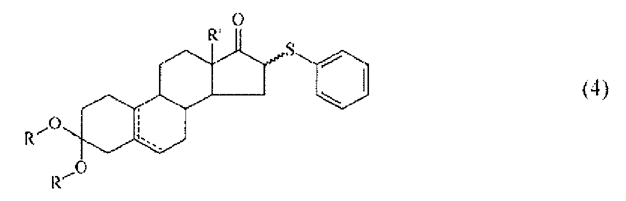 dans laquelle
R' représente un hydrogène, un méthyle ou un éthyle, et les deux groupes R représentent un même groupe divalent -(CH₂)₂-, -(CH₂)₃-, -CH₂-C(CH₃)₂-CH₂-, et la ligne en pointillé représente une double liaison 5-6 ou 5-10.

10. Composé selon la revendication 9, dans lequel R' représente un éthyle et les deux groupes R représentent tous deux -CH₂-C(CH₃)₂-CH₂-.

11. Composé de formule 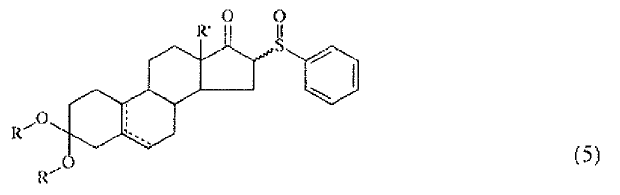 dans laquelle
R' représente un hydrogène, un méthyle ou un éthyle,
les deux groupes R représentent un même groupe divalent -(CH₂)₂-, -(CH₂)₃-, - CH₂-C(CH₃)₂-CH₂-, et
la ligne en pointillé représente une double liaison 5-6 ou 5-10.

12. Composé selon la revendication 11, dans lequel R' représente un éthyle et les deux groupes R représentent tous deux -CH₂-C(CH₃)₂-CH₂-.

13. Utilisation des composés de formule (3), (4) ou (5) pour la préparation de Δ¹⁵⁻¹⁶-cétostéroïdes de formule 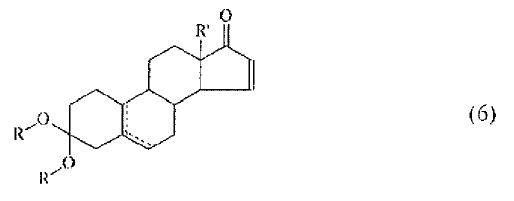 dans laquelle
R' représente un hydrogène, un méthyle ou un éthyle,
les deux groupes R représentent un même groupe divalent -(CH₂)₂-, -(CH₂)₃-, - CH₂-C(CH₃)₂-CH₂-,
et la ligne en pointillé représente une double liaison 5-6 ou 5-10.

14. Utilisation des composés de formule (3a), 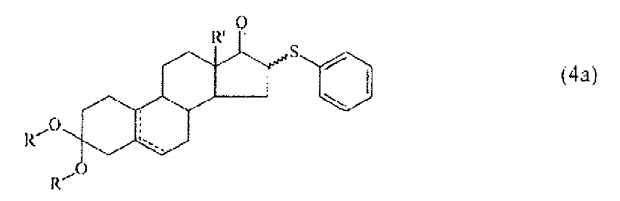 ou 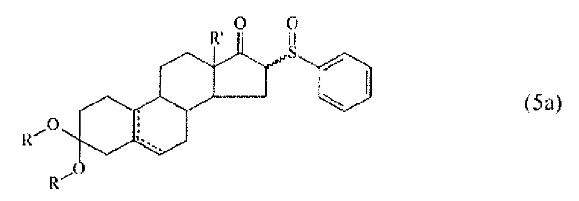 pour la préparation d'un composé de formule 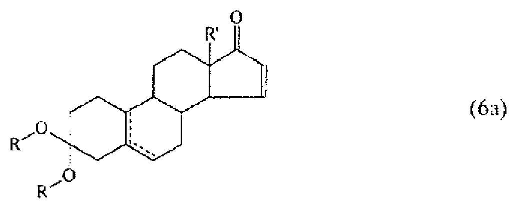 dans lequel les deux groupes R représentent tous deux -CH₂-C(CH₃)₂-CH₂-, la ligne en pointillé représente une double liaison 5-6 ou 5-10 et R' est un éthyle.

15. Utilisation de composés de formule (3a), 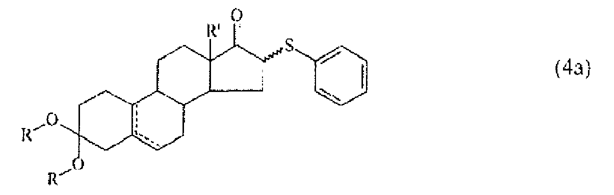 ou 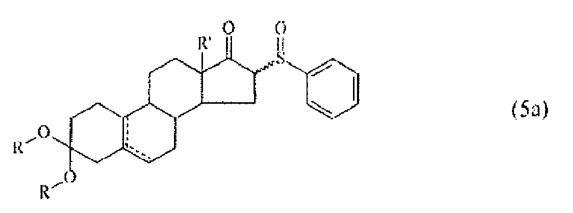 dans lesquelles les deux groupes R représentent tous deux -CH₂-C(CH₃)₂-CH₂-, la ligne en pointillé représente une double liaison 5-6 ou 5-10 et R' est un éthyle, pour la préparation de gestodène (1).
